# EUROPEAN PATENT APPLICATION

(11) **EP 4 265 652 A1**
(43) Date of publication of application: **25.10.2023**
(21) Application number: 21910478.3
(22) Date of filing: 14.12.2021
(51) Int. Cl.: C08F 30/08, A61Q 1/00, A61Q 19/10, A61Q 5/06, C08F 2/38, C07F 7/08, A61K 8/893

(54) **MONOMER COMPOSITION AND METHOD FOR PRODUCING SAME, AND COSMETIC OR COSMETIC RAW MATERIAL INCLUDING MONOMER COMPOSITION**

(30) Priority: 21.12.2020 JP 2020211245
(71) Applicant: Dow Toray Co., Ltd., Tokyo 140-8617 (JP)
(72) Inventor: AKIYAMA, Katsuhiro, Ichihara-shi Chiba 299-0108 (JP); SOUDA, Tatsuo, Ichihara-shi Chiba 299-0108 (JP); SUGIURA, Tsunehito, Ichihara-shi Chiba 299-0108 (JP)
(74) Representative: Murgitroyd & Company
(86) International application number: PCT/JP2021/046061
(87) International publication number: WO 2022/138329

(57) **Abstract**

[Problem]

To provide a monomer composition for which there is no concern about safety when used as a raw material or the like in a cosmetic material and that does not cause deterioration in quality such as gelation or the like, and to provide a method for manufacturing the monomer composition.

[Resolution Means]

A monomer composition containing a radical polymerizable monomer having a radical polymerizable organic group and an organosilicon-containing organic group in a molecule, wherein a total concentration of a polymerization inhibitor based on a total mass of the radical polymerizable monomer and the polymerization inhibitor is 130 ppm by mass or less, as well as a method for manufacturing the monomer composition.

## Description

### [TECHNICAL FIELD]

The present invention relates to a monomer composition, a method for manufacturing the same, and a cosmetic material or a cosmetic raw material containing the monomer composition.

### [BACKGROUND ART]

Use of a polymer prepared from a monomer composition containing a radical polymerizable group as a film-forming agent is known, in order to improve the water resistance and sebum resistance of cosmetic materials, in particular make-up cosmetic materials, thereby improving the cosmetic durability. In particular, an organopolysiloxane containing a radical polymerizable organic group can impart water repellency and slipperiness to a cosmetic material. However, there are cases where there is a problem with the compatibility of cosmetic raw materials to be blended and the like.

In order to solve such problems, copolymers having a carbosiloxane dendrimer structure or a siloxane macromonomer structure have been proposed. For example, Patent Document 1 proposes a copolymer based on an unsaturated monomer having a specific carbosiloxane dendrimer structure, and shows that the copolymer can be used as a film-forming agent which is excellent in blending stability with cosmetic raw materials such as an ultraviolet absorber or the like, and also excellent in water resistance and sebum resistance.

On the other hand, in order to manufacture a monomer having a complex structure such as a carbosiloxane dendrimer structure, a siloxane macromonomer structure, and the like, it is necessary to go through a plurality of steps. In particular, some raw materials in the upstream process have high reactivity, so measures are generally taken to prevent the raw materials from reacting and gelling during storage of the raw materials and during the process by adding a polymerization inhibitor, and the like.

For example, Patent Document 2 describes a silicone monomer composition including a silicone monomer containing a polymerizable group and 5 to 400 ppm of a polymerization inhibitor having a specific structure with respect to the silicone monomer. Furthermore, Patent Document 3 describes that the cosmetic composition contains a 10 ppm to 20 mass% of a polymerization inhibitor with respect to the total mass of the composition in order to increase the storage stability of the cosmetic composition.

However, it has been pointed out that some polymerization inhibitors are suspected to be mutagenic, and when used in direct contact with the human body as with cosmetic materials, the content is preferably reduced as much as possible from the viewpoint of safety. Therefore, it is desirable to separate the polymerization inhibitor from the monomer composition, but it is difficult to remove the polymerization inhibitor by means such as distillation, particularly when the raw material is an organopolysiloxane containing a radical polymerizable organic group, or the like. Specifically, although it is necessary to heat the monomer composition for distillation, there is a problem in that unintended polymerization and gelation may occur and the amount of the polymerization inhibitor cannot be reduced below a certain amount.

### [Prior Art Documents]

### [Patent Documents]

Patent Document 1: Japanese Unexamined Patent Application 2014-40512
Patent Document 2: Japanese Unexamined Patent Application 2004-115790
Patent Document 3: Japanese Unexamined Patent Application 2006-169234

### [SUMMARY OF THE INVENTION]

### [PROBLEM TO BE SOLVED BY THE INVENTION]

An object of the present invention is to solve the aforementioned problems of the conventional technology, and provide a monomer composition which has no concern about safety when used as a raw material of a cosmetic material or the like and that does not cause deterioration of quality such as gelation, and to provide a method for manufacturing the monomer composition.

### [MEANS FOR SOLVING THE PROBLEM]

As a result of conducting diligent research on the problem described above, the present inventors arrived at the present invention. In other words, an object of the present invention is achieved by a monomer composition containing a radical polymerizable monomer having a radical polymerizable organic group and an organosilicon-containing organic group in a molecule, wherein the total concentration of a polymerization inhibitor based on a total mass of the radical polymerizable monomer and the polymerization inhibitor is 130 ppm by mass or less. Furthermore, the present invention also relates to a method for manufacturing the monomer composition of the present invention, including: a step of bringing a liquid containing a radical polymerizable monomer into contact with an adsorbent material selected from the group consisting of activated carbon and alumina. The invention also relates to a cosmetic material or cosmetic raw material containing the monomer composition of the present invention.

### [EFFECTS OF THE INVENTION]

With the monomer composition of the present invention, the amount of the polymerization inhibitor is reduced to an extremely low level, so there is no concern about safety even when the monomer composition is used as a raw material for applications in direct contact with the human body, such as cosmetic materials and the like.

In addition, the method for manufacturing a monomer composition of the present invention does not include a heating step by distillation or the like. Therefore, even though the amount of the polymerization inhibitor is low, gelation or the like due to an unintended reaction will not occur, and thus a high-quality monomer composition can be provided.

### [BRIEF DESCRIPTION OF THE DRAWINGS]

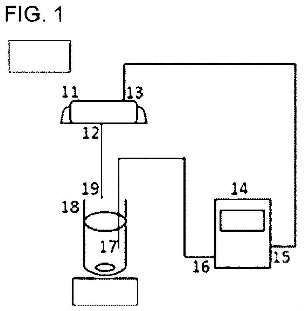

FIG. 1 schematically shows a device for separating a polymerization inhibitor in a method for manufacturing a monomer composition of an example.

### [MODE FOR CARRYING OUT THE INVENTION]

[Radical Polymerizable Monomer Having Radical Polymerizable Organic Group and Organosilicon-containing Organic Group in a Molecule]

The monomer composition of the present invention contains a radical polymerizable monomer having a radical polymerizable organic group and an organosilicon-containing organic group in a molecule.

Examples of the organosilicon-containing organic group contained in the radical polymerizable monomer include a carbosiloxane dendrimer structure and a branched or linear siloxane structure. A carbosiloxane dendrimer structure is preferred.

The carbosiloxane dendrimer structure is a chemical structure that is highly branched radially from one silicon atom, and the radical polymerizable monomer having the carbosiloxane dendrimer structure is preferably a monomer expressed by the following general formula (1): 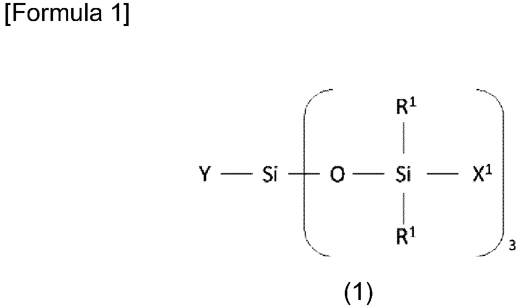 {where
Y is a radical polymerizable organic group,
R¹ is an alkyl group, an aryl group or a trimethylsiloxy group;
X¹ is a silylalkyl group expressed by the following general formula (2) when i = 1: 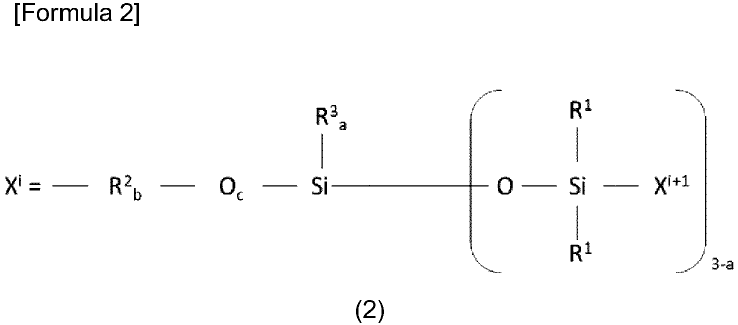 (where
   R¹ is the same group as defined for general formula (1);
   R² is an alkylene group with 2 to 10 carbon atoms;
   R³ is a group selected from the group consisting of alkoxy groups, hydroxyl groups, alkyl groups, aryl groups, and trimethylsiloxy groups;
   Xⁱ⁺¹ is a group selected from the group consisting of hydrogen atoms, alkyl groups with 1 to 10 carbon atoms, aryl groups, and the above silylalkyl groups, where i is an integer from 1 to 10, indicating the hierarchy of the silylalkyl group; a is an integer from 0 to 3; and
   (b and care 0 or 1.)}

The radically polymerizable organic group is not particularly limited so long as the organic group can be radically reacted, and specific examples include (meth)acryloxy group-containing organic groups, (meth)acrylamide group-containing organic groups, styryl group-containing organic groups, and alkenyl groups with 2 to 10 carbon atoms. An organic group containing a (meth)acryloyl group is preferable. Examples of the radical polymerizable organic group include organic groups expressed by the following general formula. 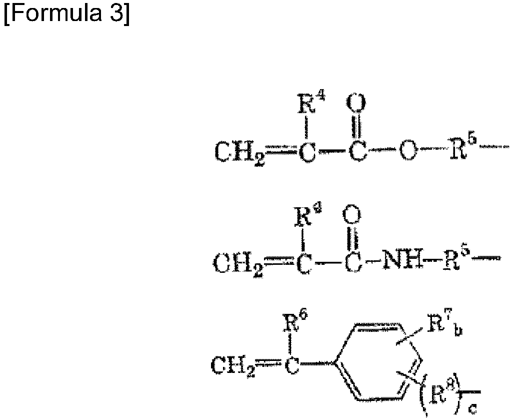 (where R⁴ and R⁶ are hydrogen atoms or methyl groups, R⁵ and R⁸ are alkylene groups with 1 to 10 carbon atoms, and R⁷ is an alkyl group with 1 to 10 carbon atoms. b is an integer from 0 to 4, and c is 0 or 1).

Examples of such a radical polymerizable organic group include acryloxymethyl groups, 3-acryloxypropyl groups, methacryloxymethyl groups, 3-methacryloxypropyl groups, 4-vinylphenyl groups, 3-vinylphenyl groups, 4-(2-propenyl) phenyl groups, 3-(2-propenyl) phenyl groups, 2-(4-vinylphenyl)ethyl groups, 2-(3-vinylphenyl)ethyl groups, vinyl groups, allyl groups, methallyl groups, and 5-hexenyl groups.

In the general formulas (1) and (2), R¹ is an alkyl group, an aryl group or a trimethylsiloxy group, preferably a methyl group or a phenyl group, and particularly preferably a methyl group.

In general formula (2), R² is an alkylene group with 2 to 10 carbon atoms, and ethylene groups, methylethylene groups, hexylene groups, 1-methylpentylene groups, and 1,4-dimethylbutylene groups are preferred.

In general formula (2), R³ is a group selected from the group consisting of alkoxy groups, hydroxyl groups, alkyl groups, aryl groups, and trimethylsiloxy groups, and examples of the alkyl group include alkyl groups with 1 to 10 carbon atoms.

In general formula (2), Xⁱ⁺¹ is a hydrogen atom or a group selected from the group consisting of alkyl groups with 1 to 10 carbon atoms, aryl groups, and the abovementioned silylalkyl groups. i is an integer from 1 to 10, and indicates the hierarchical number of the silylalkyl group, or in other words, the number of repetitions of the silylalkyl group. Thus, when the hierarchical number is 1, the radical polymerizable monomer is expressed by the following general formula.

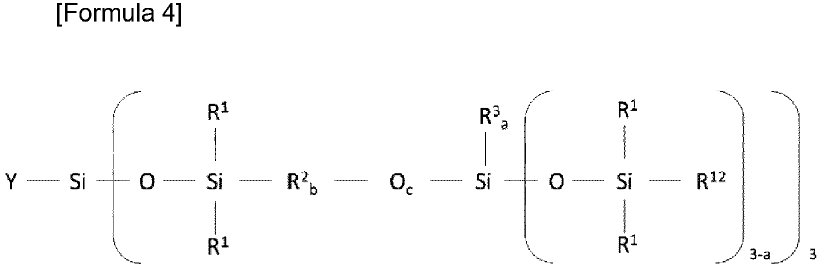

(In the formula, Y, R¹, R², and R³ are the same groups as defined for the general formulas (1) and (2), and R¹² is a hydrogen atom or the same groups as R¹ above.
a is the same number as defined for general formula (1), but the average total number of a in a molecule is 0 to 7.)
When the hierarchical number is 2, the radical polymerizable monomer is expressed by the following general formula.

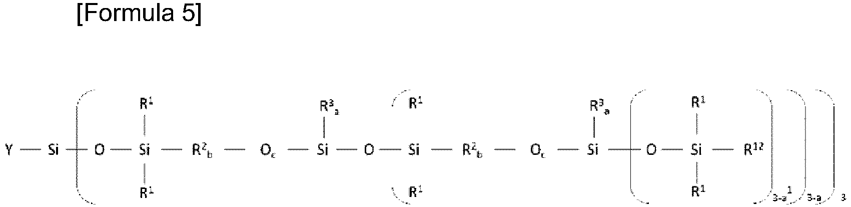

(In the formula, Y, R¹, R², R³, and R¹² are the same groups as defined for general formulas (1) and (2).
a and a¹ are the same numbers as defined for general formula (1), but the average total number of a and a' in a molecule is 0 to 25.)

Examples of the radical polymerizable monomer having the carbosiloxane dendrimer structure of the present component include the monomers expressed by the following average compositional formulas.

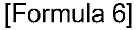

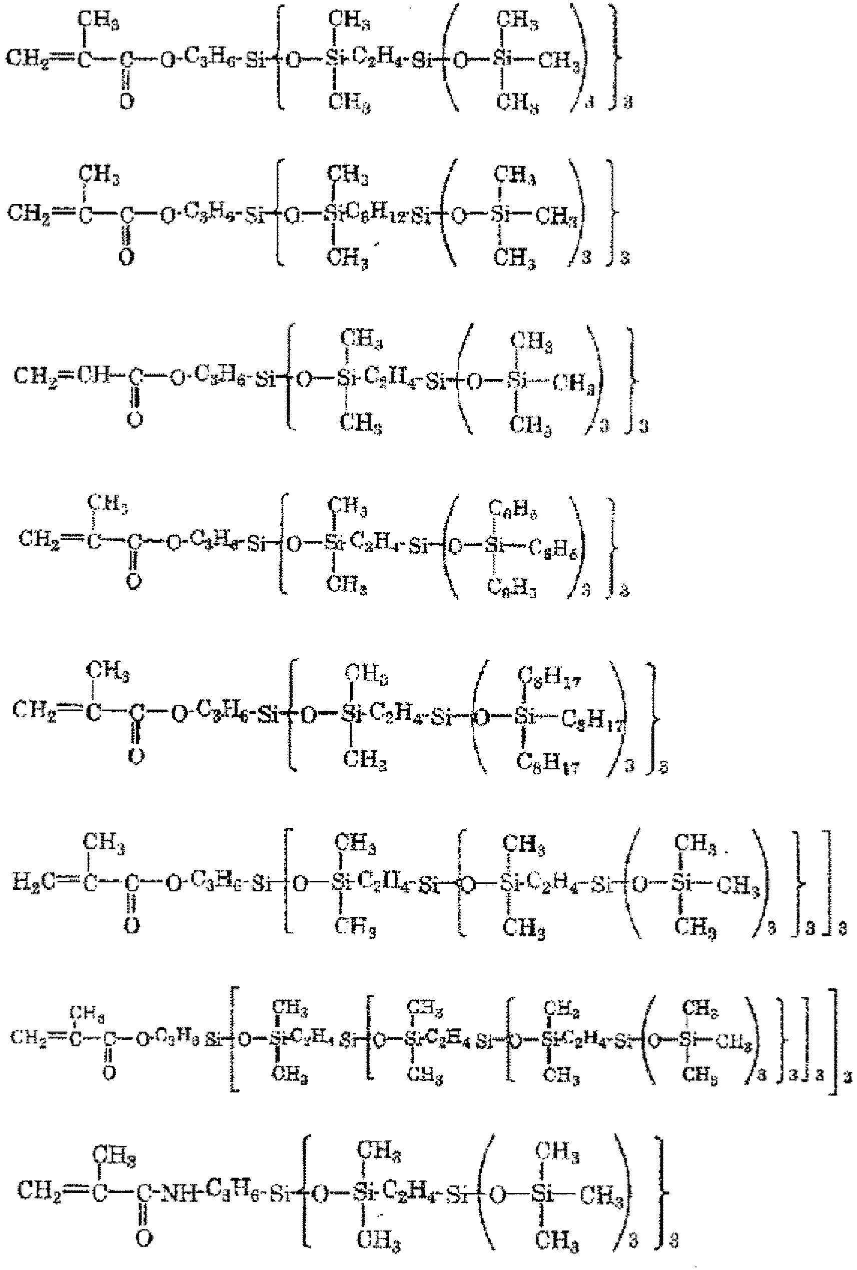

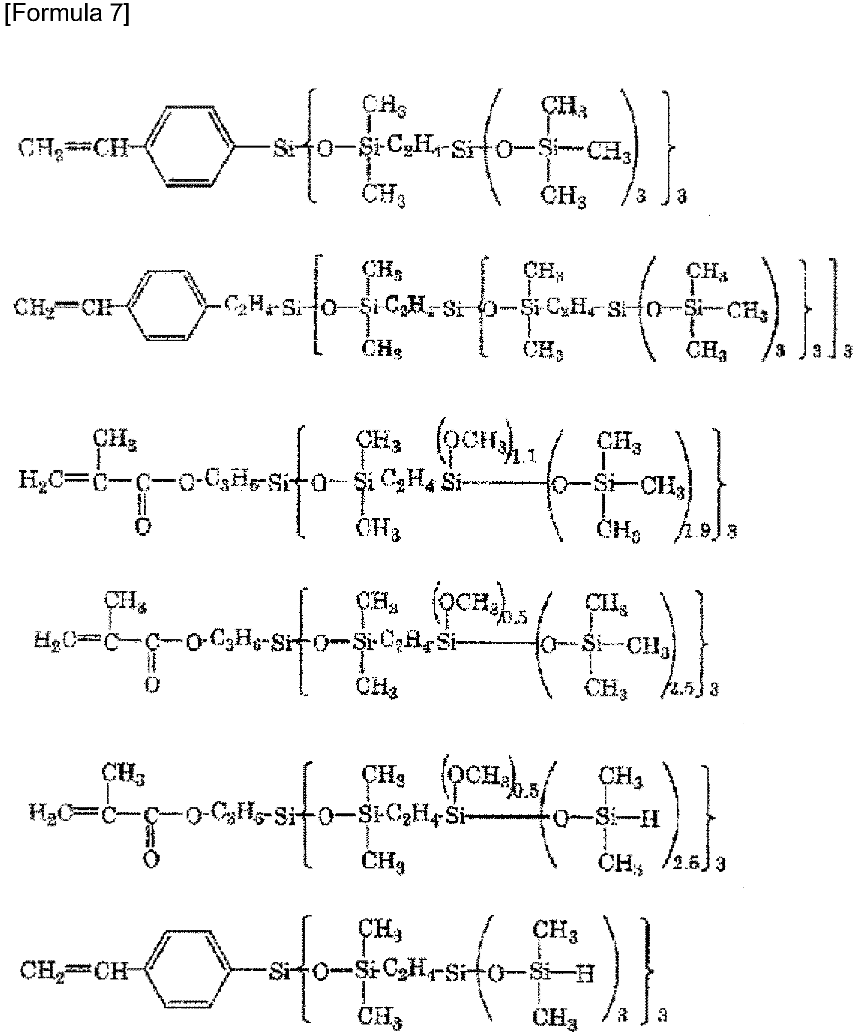

Such carbosiloxane dendrimers can be produced in accordance with a method of manufacturing a branched siloxane-silalkylene copolymer described in Japanese Unexamined Patent Application H11-1530. Examples can be manufactured by a hydrosilylation reaction between an organosilicon compound containing an alkenyl group and a silicon compound containing a silicon atom-bonded hydrogen atom expressed by the following general formula: 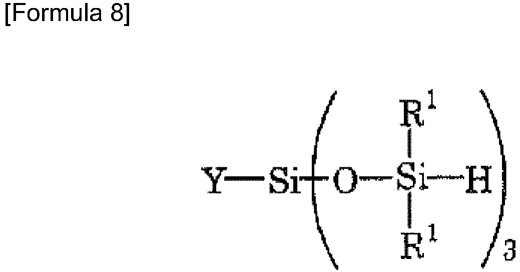 (in the formula, R¹ and Y are the same groups as defined for general formula (1)).
Examples of the silicon compound represented by the above formula include 3-methacryloxypropyl tris(dimethylsiloxy)silane, 3-acryloxypropyl tris(dimethylsiloxy) silane, and 4-vinylphenyl tris(dimethylsiloxy)silane. As the alkenyl group-containing organosilicon compound, vinyltris(trimethylsiloxy) silane, vinyltris(dimethylphenylsiloxy)silane, and 5-hexenyl tris(trimethylsiloxy) silane are used. Note that the hydrosilylation reaction is preferably performed in the presence of a transition metal catalyst such as chloroplatinic acid or a platinum vinylsiloxane complex, or the like.

Radical polymerizable monomers with a branched or linear siloxane structure include organopolysiloxanes containing M units expressed by R^{a}₃SiO_{1/2} or R^{a}₂R^{b}SiO_{1/2}, D units expressed by R^{a}₂SiO_{2/2} or R^{a}R^{b}SiO_{2/2}, T units expressed by R^{a}SiO_{3/2} or R^{b}SiO_{3/2}, and Q units expressed by SiO_{4/2}, in any ratio. Herein, R^{a} is an alkyl group, an aryl group, an aralkyl group, or a group in which some or all of the hydrogen atoms of these groups are replaced with halogen atoms, and R^{b} is a radical polymerizable organic group. Radical polymerizable monomers with branched or linear siloxane structures are preferably macromonomers with a relatively large weight average molecular weight.

R^{a} is an alkyl group, an aryl group, an aralkyl group, or a group in which some or all of the hydrogen atoms of these groups are substituted with halogen atoms. An alkyl group with 1 to 10 carbon atoms is preferred as the alkyl group, with examples including methyl groups, ethyl groups, propyl groups, butyl groups, pentyl groups, isopropyl groups, isobutyl groups, cyclopentyl groups, and cyclohexyl groups. Examples of aryl groups include phenyl groups and naphthyl groups. Moreover, examples of aralkyl groups include benzyl groups and phenethyl groups. Examples of groups in which some or all of the hydrogen atoms are substituted with halogen atoms include fluorine-substituted alkyl groups such as trifluoropropyl groups, heptadecafluorodecyl groups, and the like.

R^{b} is a radical polymerizable organic group, which is not particularly limited, so long as the organic group can be radically reacted, and can be the same groups as defined for the carbosiloxane dendrimer structure. In other words, examples include (meth)acryloxy group-containing organic groups, (meth)acrylamide group-containing organic groups, styryl group-containing organic groups, and alkenyl groups with 2 to 10 carbon atoms.

Radical polymerizable monomers having such a branched or linear siloxane structure can be synthesized using raw materials and methods well known to a person skilled in the art. Examples include those monomers obtained by reacting an organosiloxane having a silanol group in the molecule with an organochlorosilane compound in the presence of a base. The organochlorosilane compound is not particularly limited so long as it has a radical polymerizable organic group, and examples include 3-methacryloxypropyl dimethylchlorosilane and 3-methacryloxypropyl dichloromethylsilane.

### [Polymerization Inhibitor]

The monomer composition of the present invention contains the polymerization inhibitor at a concentration where the total concentration of the polymerization inhibitor relative to the total mass of the radical polymerizable monomer and the polymerization inhibitor is 130 ppm by mass or less. Preferably, the total concentration of the polymerization inhibitor relative to the total mass of the radical polymerizable monomer and polymerization inhibitor is 110 ppm by mass or less, more preferably 90 ppm by mass or less, and even more preferably 70 ppm by mass or less.

The polymerization inhibitor includes one or more types selected from hindered phenol-based polymerization inhibitors, hydroquinone-based polymerization inhibitors, and catechol-based polymerization inhibitors. Examples of hindered phenol-based polymerization inhibitors include dibutylhydroxytoluene, 2,6-di-tert-butylphenol, 2,4-di-tert-butylphenol, 2-tert-butyl-4,6-dimethylphenol, 2,6-di-tert-butyl-4-methylphenol, and 2,4,6-tri-tert-butylphenol. Examples of hydroquinone-based polymerization inhibitors include methylhydroquinone, ethylhydroquinone, propylhydroquinone, tert-butylhydroquinone, 2,5-di-tert- butylhydroquinone, hydroquinone monomethyl ether, p-benzoquinone, and 2,5-diphenylparabenzoquinone. Examples of catechol-based polymerization inhibitors include catechol, 2-methylcatechol, 3-methylcatechol, 4-methylcatechol, 2-ethylcatechol, 3 ethylcatechol, 4-ethylcatechol, 2-propylcatechol, 3-propylcatechol, 4-propylcatechol, 2-n-butylcatechol, 3-n-butylcatechol, 4-n-butylcatechol, 2-tert butylcatechol, 3-tert-butylcatechol, 4-tert-butylcatechol, and 3,5-di-tert-butylcatechol. In particular, the monomer composition of the present invention includes one or more types selected from dibutylhydroxytoluene (BHT), methyl hydroquinone (MEHQ), and 4-tert-butylcatechol (TBC). These hindered phenol-based phenol polymerization inhibitors, hydroquinone-based polymerization inhibitors, and catechol-based polymerization inhibitors have been reported to be suspected to be mutagenic in some cases, and it is desirable to reduce the amount in order to reduce the potential risk.

### [Method for Manufacturing Monomer Composition]

Furthermore, in one embodiment, the present invention also relates to a method for manufacturing the monomer composition of the present invention, including: a step of bringing a liquid containing a radical polymerizable monomer into contact with an adsorbent material selected from the group consisting of activated carbon and alumina. Generally, the crude monomer composition that is obtained is purified by distillation, or the like. In such cases, unintended polymerization and gelation may occur, and the quality of the monomer composition may be problematic. Therefore, the polymerization inhibitors are used without sufficiently reducing the concentration.

The activated carbon used in the manufacturing method of the monomer composition of the present invention is not particularly limited, and is manufactured by activating carbon substances by reacting them with a gas or chemical substance at high temperatures. The adsorbent material can be used alone or in combination with adsorbent materials other than alumina and activated carbon, such as zeolite, silica, aluminum silicate, and the like. Activated carbon is preferred as the adsorbent material.

Contact between the adsorbent material and the liquid containing the radical polymerizable monomer can be made without solvent or in the presence of a solvent. The temperature and pressure at which the process of contacting the adsorbent material with the liquid containing the radical polymerizable monomer is performed are not particularly limited, but can be performed at 0°C to 200°C, preferably 5°C to 150°C, more preferably 10°C to 100°C, at atmospheric pressure.

The method of bringing the adsorbent material into contact with the liquid containing the radical polymerizable monomer is not particularly limited, but can be performed by a fixed bed method in which the adsorbent material is packed in a container and the liquid containing the radical polymerizable monomer is continuously supplied, a batch method in which stirring and mixing and solid-liquid separation are each performed in batch operations, a moving layer method in which the adsorbent material is a moving layer and passed through the liquid containing the radical polymerizable monomer, or a fluidized bed method, in which a solid layer of adsorbent material is fluidized by the liquid containing the radical polymerizable monomer. The process of bringing the adsorbent material into contact with the liquid containing the radical polymerizable monomer is particularly preferably performed in a fixed bed system, and continuously supplying the liquid containing the radical polymerizable monomer to the fixed bed of activated carbon is more preferable.

In the case of the fixed bed method, the liquid containing the radical polymerizable monomer can be passed through the fixed bed of adsorbent material only once, or the liquid containing the radical polymerizable monomer can be circulated by supplying the outlet liquid of the fixed bed of adsorbent material back into the inlet. The circulating method is preferred because this method makes it easier to ensure the contact time between the liquid and the adsorbent material.

In a fixed bed system, the feed flow rate of the liquid containing the radical polymerizable monomer can be appropriately determined according to the type and concentration of the polymerization inhibitor and adsorbent material. The feed flow rate of the liquid containing the radical polymerizable monomer is not particularly limited, but can be calculated as the feed flow rate of the liquid divided by the mass of the adsorbent material (g-AD), and the flow rate can be 0.1 g/(min·g-AD) to 100 g/(min·g-AD), preferably 0.5 g/(min·g-AD) to 50 g/(min·g-AD), and more preferably 1.0 g/(min·g-AD) to 10 g/(min.g-AD). The feed flow rate is preferably within an appropriate range to ensure sufficient contact time with the adsorbent material and proper removal of the polymerization inhibitor.

In a fixed bed system, if the liquid containing the radical polymerizable monomer is circulated, the circulation frequency can also be appropriately determined according to the type and concentration of the polymerization inhibitor and adsorbent material. The circulation frequency is not particularly limited, but can be 0.1 to 20 times/hour, preferably 0.5 to 15 times/hour, and more preferably 1 to 10 times/hour. By setting the circulation frequency within the range above, the concentration of the polymerization inhibitor in the liquid becomes more uniform, which is preferable for proper removal of the polymerization inhibitor.

In a batch-type system, the stirring and mixing time of the liquid containing the radical polymerizable monomer can be appropriately determined according to the type and concentration of the polymerization inhibitor and adsorbent material. The time for stirring and mixing the liquid containing the radical polymerizable monomer is not particularly limited, but can be 0.5 to 30 hours, preferably 1 to 20 hours, and more preferably 3 to 10 hours.

The method of manufacturing the monomer composition or the present invention may include a step of bringing the adsorbent material into contact with the liquid containing the radical polymerizable monomer and then adding a polymerization inhibitor that does not have a risk of mutagenicity, or the like, preferably a polymerization inhibitor other than BHT, MEHQ and TBC, such as tetra(di-t-butyl hydroxyhydrosilicate) pentaerythryl and other silicic acid derivatives, alpha-tocopherol, propyl gallate, and the like.

### [Cosmetic Material]

In one embodiment, the monomer composition of the present invention can be used as a cosmetic raw material or a cosmetic material, and may be included as a cosmetic raw material or cosmetic material in a copolymer that is polymerized with other radical polymerizable monomers. Cosmetic raw materials and cosmetic materials may be compositions containing at least one type selected from the group consisting of: (A) oils, (B) alcohols, (C) surfactants, (D) powders or colorants, (E) gelling agents or thickeners, (F) organically modified clay minerals, (G) silicone resins, (H) silicone gums, (I) silicone elastomers, (J) organically modified silicones, (K) ultraviolet light blocking components, and (L) water-soluble polymers. Note that these specific examples are common to the components disclosed in the aforementioned Patent Document 1 (Japanese Unexamined Patent Application 2014-40512) and the like.

### (A) Oils

Examples of the oils include animal oils, plant oils, synthetic oils, and the like, that are generally used in cosmetic materials. The oil may be a solid, semisolid, or liquid, and may be non-volatile, semi-volatile, or volatile. The oil is used to impart lubricity to the skin or hair, soften the skin and impart a moisturized feeling. The oil can also be used to dilute the copolymer of the present invention to obtain a copolymer composition, and in particular, is a liquid at 5 to 100°C. The oil is preferably at least one type selected from silicone-based oils (A1) and organic oils (A2), and the type and viscosity, or the like of these oils can be appropriately selected according to the type of cosmetic material and the application. These oils are added to the cosmetic raw material or cosmetic material of the present invention at the same time as the monomer composition.

### (B) Alcohols

One or more polyhydric alcohol and/or lower monohydric alcohol can be used as the alcohol. Examples of the lower alcohols include ethanol, isopropanol, n-propanol, t-butanol, sec-butanol, and the like, but ethanol is preferable. Examples of polyhydric alcohols include dihydric alcohols such as 1,3-propanediol, 1,3-butylene glycol, 1,2-butylene glycol, propylene glycol, trimethylene glycol, tetramethylene glycol, 2,3-butylene glycol, pentamethylene glycol, 2-butene-1,4-diol, dibutylene glycol, pentyl glycol, hexylene glycol, octylene glycol, and the like; trihydric alcohols such as glycerin, trimethylolpropane, 1,2,6-hexanetriol, and the like; tetrahydric or higher polyhydric alcohols such as pentaerythritol, xylitol, and the like; and sugar alcohols such as sorbitol, mannitol, maltitol, maltotriose, sucrose, erythritol, glucose, fructose, starch degradation products, maltose, xylitose, starch-degrading sugar-reducing alcohols, and the like. Other examples in addition to these low molecular weight polyhydric alcohols include diethylene glycol, dipropylene glycol, triethylene glycol, polypropylene glycol, tetraethylene glycol, diglycerin, polyethylene glycol, triglycerin, tetraglycerin, polyglycerin, other polyhydric alcohol polymers, and the like. Of these, ethanol, 1,3-propanediol, 1,3-butylene glycol, sorbitol, dipropylene glycol, glycerin, and polyethylene glycol are particularly preferable.

The cosmetic material or the cosmetic raw material containing the monomer composition of the present invention or a copolymer obtained therefrom may contain (C) a surfactant as an optional component. The surfactant (C) can be one or more types of surfactants, used in combination according to the objective, selected from the group consisting of (C1) silicone-based surfactants, (C2) anionic surfactants, (C3) cationic surfactants, (C4) nonionic surfactants, (C5) amphoteric surfactants, and (C6) semipolar surfactants.

Note that examples of (C1) silicone-based surfactants include polyglyceryl modified silicones, diglyceryl modified silicones, glyceryl modified silicones, sugar modified silicones, fluoropolyether modified silicones, polyether modified silicones, carboxylic acid modified silicones, linear silicone polyether block copolymers (such as polysilicone-13 and the like), long-chain alkyl-polyether co modified silicones, polyglyceryl modified silicone elastomers, diglyceryl modified elastomers, glyceryl modified elastomers, and polyether modified elastomers, and the like. Furthermore, a silicone-based surfactant having an alkyl branch, linear silicone branch, or siloxane dendrimer branch, or the like can be appropriately used in the silicone or elastomer as necessary, at the same time as a hydrophilic group. Commercially available products include SH 3771 M, SH 3772 M, SH 3773 M, SH 3775 M, BY 22-008M, BY 11-030, ES - 5373 FORMULATION AID, ES - 5612 FORMULATION AID, ES - 5300 FORMULATION AID, and ES - 5600 SILICONE GLYCEROL EMULSIFIER (all are available from Dow Toray Co., Ltd.).

The amount of the surfactant (C) that is added to the cosmetic material or the cosmetic raw material containing the monomer composition of the present invention or a copolymer obtained therefrom is not particularly limited. However, in order to stabilize the emulsion or dispersion, the surfactant (F) can be added to the emulsion composition or dispersion composition in a range of 0.05 to 90 mass%, preferably from 0.1 to 50 mass%, and even more preferably from 0.5 to 25 mass%, based on the weight of the composition.

### (D) Powder or Colorant

The cosmetic material or the cosmetic raw material containing the monomer composition of the present invention or a copolymer obtained therefrom can further contain powders or colorants, particularly any powders used in cosmetic products (including powders and pigments used as colorants). Any powder or colorant can be used as long as the powder or colorant is one that is used in ordinary cosmetic materials, regardless of the shape (such as spherical, a rod-shape, needle-shape, plate-shape, sheet-shape, indefinite shape, spindle-shape, bowl-shape, raspberry shape, and the like), the particle size (such as in an aerosol form, microparticle form, pigment class, and the like) and the particle structure (such as porous, non-porous, secondary aggregation, and the like) of the powder or colorant, and when these powders and/or colorants are compounded as a pigment, one or more types selected from inorganic pigment powders, organic pigment powders, and resin powders having an average particle diameter in the range of 1 nm to 20 µm are preferably added.

Specific examples of the powder or colorant include inorganic powders, organic powders, surfactant metal salt powders (metal soaps), colored pigments, pearl pigments, metal powder pigments, silicone elastomer powders, and the like. Composites of these can also be used. Note that these powders or colorants include those which function as ultraviolet light blocking components.

Furthermore, it is particularly preferable that some or all of these powders or colorants are subjected to a water-repellent treatment. Such a treatment allows for stable compounding into the oil phase. Furthermore, these powders or colorants may be combined together, and powders and colorants that have been subjected to a surface treatment using a general oil, silicone compounds other than the organopolysiloxane copolymer of the present invention, or fluorine compounds or surfactants, or the like can also be used.

Furthermore, some or all of these powders or colorants can be subjected to a hydrophilizing treatment. Thereby, the powder or colorant can be added in an aqueous phase.

Furthermore, some or all of these powders or colorants can be subjected to a hydrophobizing treatment or hydrophilizing treatment. Through such treatments, emulsification characteristics can be imparted to the powder itself. Examples of commercially available products include MZY-500SHE available from Tayca Corporation and the like.

One type or two or more types of the powder or colorant (D) in the cosmetic material or the cosmetic raw material containing the monomer composition of the present invention or a copolymer obtained therefrom may be used as necessary, and the added amount is not particularly limited. Furthermore, the amount of the powder or colorant can be added within a range of 0.1 to 99.5 mass%, and preferably 1 to 99 mass%, of the total composition or cosmetic material. In particular, the compounded amount for a powdered solid cosmetic material is preferably within the range of 80 to 99 mass% of the entire cosmetic material.

### (E) Gelling agent or Thickener

The gelling agent is preferably an oil-soluble gelling agent, and specific examples include metal soaps such as aluminum stearate, magnesium stearate, zinc myristate, and the like; amino acid derivatives such as N-lauroyl-L-glutamic acid, α,γ-di-n-butylamine, and the like; dextrin fatty acid esters such as dextrin palmitate, dextrin stearate, dextrin 2-ethylhexanoic acid palmitate, and the like; sucrose fatty acid esters such as sucrose palmitate, sucrose stearate, and the like; and benzylidene derivatives of sorbitol such as monobenzylidene sorbitol, dibenzylidene sorbitol, and the like. One or two or more of these can be used as necessary.

### (F) Organically Modified Clay Minerals

Examples of the organically modified clay minerals include dimethylbenzyldodecylammonium montmorillonite clay, dimethyldioctadecylammonium montmorinite clay, dimethylalkylammonium hectorite, benzyldimethyl stearylammonium hectorite, distearyldimethylammonium aluminum magnesium chloride-treated magnesium aluminum silicate, and the like. Commercially available products thereof include Benton 27 (hectorite treated with benzyl dimethyl stearyl ammonium chloride, available from National Lead Co.), Benton 38 (hectorite treated with distearyl dimethyl ammonium chloride, available from National Lead Co.), and the like.

### (G) Silicone Resin

The silicone resin is an organopolysiloxane having a highly branched structure, a net-like structure, or a cage-like structure, and is in a liquid state or a solid state at ambient temperature, and may be any silicone resin commonly used in cosmetic materials as long as the silicone resin does not contradict the object of the present invention. Examples of solid silicone resins include MQ resin, MDQ resin, MTQ resin, MDTQ resin, TD resin, TQ resin, and TDQ resin made from optional combinations of triorganosiloxy units (M unit) (organo groups are methyl groups only, or are methyl groups and vinyl groups or phenyl groups), diorganosiloxy units (D unit) (organo groups are methyl groups only, or are methyl groups and vinyl groups or phenyl groups), monoorganosiloxy units (T unit) (organo groups are methyl groups, vinyl groups, or phenyl groups), and siloxy units (Q unit). Furthermore, examples include trimethyl siloxysilicate, polyalkyl siloxysilicate, trimethyl siloxysilicate containing dimethylsiloxy units, and alkyl (perfluoroalkyl) siloxysilicate. It is particularly preferable that these silicone resins are oil-soluble and can be dissolved in (A).

The silicone resin forms a uniform film when applied to skin, hair, or the like, and provides a protective effect against drying and low temperature. Furthermore, the silicone resin having these branching units tightly adheres to the skin, hair, and the like, and can impart luster and a transparent feel to skin, hair, and the like.

### (H) Silicone Gum

In the present invention, ultra-high viscosity organopolysiloxanes, referred to as silicone gums, having a viscosity of greater than or equal to 1,000,000 mm²/s can also be used as silicone oils. Silicone gum is a linear diorganopolysiloxane with an ultra-high degree of polymerization, and is also referred to as a raw silicone rubber or an organopolysiloxane gum. Silicone gum has a measurable degree of plasticity due to the high degree of polymerization, and is thereby distinguished from the silicone-based oils described above. Additionally, with the present invention, one type or a combination of two or more types of silicone gums can be used as necessary. The silicone gum can be added to the cosmetic material or the cosmetic raw material containing the monomer composition of the present invention or a copolymer obtained therefrom, either as is, or as a liquid gum dispersion (oil dispersion of silicone gum) with the silicone gum dispersed in an oily silicone.

Silicone gums have an ultra-high degree of polymerization, and therefore form a protective film having excellent residual properties on skin and hair and excellent air permeability. Thus, silicone gum is a component that can impart luster and gloss to particularly skin and hair, and can impart tension and a resilient texture to the entire skin and hair both during and after use.

The added amount of the silicone gum is, for example, in a range of 0.05 to 30 mass%, and preferably from 1 to 15 mass%, based on the total amount of cosmetic material. Note that the silicone gum is easy to compound if used as an emulsion composition prepared in a pre-emulsification step (also including emulsion polymerization), and can be stably added to the cosmetic material or the cosmetic raw material containing the monomer composition of the present invention or a copolymer obtained therefrom. If the compounded amount of the silicone gum is less than the abovementioned lower limit, the effect of imparting gloss to the skin and hair may be insufficient.

### (I) Silicone Elastomer

The silicone elastomer can be added to the cosmetic material or cosmetic raw material in any form in accordance with the purpose thereof, but in particular, in addition to the silicone elastomer powder described in the aforementioned "(D) Powder", adding the silicone elastomer as a crosslinkable organopolysiloxane is preferable. The silicone elastomer powder can also be used in the form of an aqueous dispersion liquid in the cosmetic material or the cosmetic raw material containing the monomer composition of the present invention or a copolymer obtained therefrom. Examples of commercially available products of such an aqueous dispersion liquid include BY29-129 and PF-2001 PIF Emulsion available from Dow Toray Co., Ltd. and the like. By compounding these silicone elastomer powders in the form of a water-based dispersion (= suspension), the feel of use of the cosmetic material or the cosmetic raw material containing the monomer composition of the present invention or a copolymer obtained therefrom can be further improved, and thus such silicone elastomer powders are extremely useful.

Examples of the crosslinkable organopolysiloxane include non-emulsifying organopolysiloxanes having a structure in which an organopolysiloxane chain is three-dimensionally crosslinked by a reaction with a crosslinkable component or the like, but not having a hydrophilic moiety such as a polyoxyalkylene unit or the like is preferable. Such crosslinkable organopolysiloxanes can be used without limitation irrespective of physical morphologies such as dilutions and properties, and of the preparation method, and the like, and α,ω-diene crosslinked silicone elastomers described in US Patent No. 5,654,362 (commercially available products include DOWSIL 9040 Silicone Elastomer Blend, DOWSIL 9041 Silicone Elastomer Blend, DOWSIL 9045 Silicone Elastomer Blend, and DOWSIL 9046 Silicone Elastomer Blend, available from The Dow Chemical Company, USA) are particularly preferred. Crosslinkable organopolysiloxanes that are fluid at room temperature can also be suitably used, and examples include DOWSIL 3901 LIQUID SATIN BLEND (The Dow Chemical Company, USA) and the like.

### (J) Organically Modified Silicone

The organically modified silicone is preferably lipophilic. Specific examples include, in addition to the above, amino modified silicones, amino polyether modified silicones, epoxy modified silicones, carboxyl modified silicones, amino acid modified silicones, carbinol modified silicones, acrylic modified silicones, phenol modified silicones, amidoalkyl modified silicones, amino glycol modified silicones, and alkoxy modified silicones. Furthermore, higher alkyl modified silicones, alkyl modified silicone resins, and polyamide modified silicone resins that are particularly preferable as organically modified silicone can be used.

### (K) Ultraviolet Light Blocking Component

The ultraviolet light blocking component includes inorganic ultraviolet light blocking components and organic ultraviolet light blocking components. If the cosmetic material or the cosmetic raw material containing the monomer composition of the present invention or a copolymer obtained therefrom is for use in a sunscreen, at least one inorganic or organic component is preferably included, and particularly an organic ultraviolet light blocking component. In particular, it is preferable to use a combination of an inorganic-based and an organic-based ultraviolet light blocking component, and it is more preferable to use a combination of an ultraviolet light blocking component corresponding to UV-A and an ultraviolet light blocking component corresponding to UV-B.

### (L) Water-Soluble Polymer

On the other hand, the cosmetic material or the cosmetic raw material containing the monomer composition of the present invention or a copolymer obtained therefrom can be an aqueous or emulsion composition containing a large amount of water-soluble components, and a water-soluble polymer (L) may be, and is preferably, added in accordance with the dosage form thereof. As the water-soluble polymer, one or more water-soluble polymer can be used.

Other components ordinarily used in cosmetic materials can be added to the cosmetic material or the cosmetic raw material containing the monomer composition of the present invention or a copolymer obtained therefrom, within a range that does not hinder the effect of the present invention, and examples of other such components include: organic resins, moisturizing agents, antiseptic agents, antimicrobial agents, perfumes, salts, antioxidants, pH adjusting agents, chelating agents, refreshing agents, anti-inflammatory agents, skin beautifying components (skin lightening agents, cell activating agents, rough skin improving agents, circulation promoters, skin astringents, anti-seborrheic agents, and the like), vitamins, amino acids, nucleic acids, hormones, inclusion compounds, and the like. These specific examples are common with, but not limited to, those examples specifically disclosed in paragraphs [0100] to [0113] and the like of Japanese Unexamined Patent Application 2011-149017 A.

The cosmetic material or the cosmetic raw material containing the monomer composition of the present invention or a copolymer obtained therefrom can contain a natural plant extract component, a seaweed extract component, or an herbal medicine component, in accordance with the purpose thereof. Two or more types of these components may be compounded. These specific examples are common with, but not limited to, those examples specifically disclosed in paragraph [0115] and the like of Japanese Unexamined Patent Application 2011-149017 A.

Depending on the purposes thereof, the cosmetic material or the cosmetic raw material containing the monomer composition of the present invention or a copolymer obtained therefrom may contain a solvent such as a light isoparaffin, ether, LPG, N-methylpyrrolidone, alternative chlorofluorocarbon, or the like, in addition to water such as purified water, mineral water, or the like.

Furthermore, at least one type of component selected from the group consisting of acrylic silicone dendrimer copolymers and alkyl modified silicone resin waxes may be used in the cosmetic material or the cosmetic raw material containing the monomer composition of the present invention or a copolymer obtained therefrom. These components are film-forming components similar to the copolymer obtained from the monomer composition of the present invention, but these components are not components having washability. Therefore, these components are preferably compounded within a range that does not impair the technical effects of the present invention.

As acrylic silicone dendrimer copolymers, for example, vinyl polymers having a carbosiloxane dendrimer structure at a side chain as described in Japanese Unexamined Patent Application 4009382 B (Japanese Unexamined Patent Application 2000-063225 A) are particularly preferred. Examples of commercially available products include FA 4001 CM Silicone Acrylate and FA 4002 ID Silicone Acrylate available from Dow Toray Co., Ltd. and the like.

As the alkyl modified silicone resin wax, for example, silsesquioxane resin wax described in Japanese PCT Application 2007-532754 T is preferable.

The cosmetic material or the cosmetic raw material containing the monomer composition of the present invention or a copolymer obtained therefrom may be in any form, such as liquid, emulsion, cream, solid, paste, gel, powder, multilayer, mousse, or spray.

The copolymer obtained from the monomer composition of the present invention can form a film that excels in washing performance while also having water resistance and sebum resistance, on the skin or hair, and thus a cosmetic material that provides these functional films can be designed.

The cosmetic material containing the monomer composition of the present invention or a copolymer obtained thereby can be used in specific products, including skin cleansing products, skin care products, make-up products, antiperspirant products, ultraviolet light blocking products, and other skin cosmetic products; hair washing agent products, hair dressing products, hair coloring products, hair grower products, hair-rinse products, hair conditioner products, hair treatment products, and other hair cosmetic products; bath cosmetic products; hair growth agents, hair tonics, and analgesics, fungicides, anti-inflammatory agents, refreshers, and skin aging inhibitors, but there is no limitation thereto. The monomer composition of the present invention or copolymers obtained therefrom come into direct contact with the human body, particularly when used as cosmetic products for skin and hair. Therefore, components with health risks, such as polymerization inhibitors that may be mutagenic, or the like were required to be removed as much as possible. The monomer composition of the present invention satisfies these requirements, and the amount of polymerization inhibitor has been greatly reduced compared to conventional products. Note that specific examples of cosmetic products for skin and hair are common to the various cosmetic products disclosed in the aforementioned Patent Document 1 (Japanese Unexamined Patent Application 2014-40512) and the like. Furthermore, the monomer composition according to the present invention or a copolymer obtained therefrom can also be used to replace some or all of the components in a composition of cosmetic materials containing acrylic copolymers with organosilicon-containing organic groups selected from known carbosiloxane dendrimer structures and branched or linear siloxane structures.

### [EXAMPLES]

The present invention will be described in detail below based on examples, but the present invention is not limited to the following examples.

### [Measuring Concentration of Polymerization Inhibitors]

A 0.25 g sample was diluted 20-fold in tetrahydrofuran (Fujifilm Wako Pure Chemicals Corporation, for high-performance liquid chromatography, no stabilizer included), and 20 µL was injected into a high-performance liquid chromatography analyzer (SHIMAZU Prominence-iLC-2030C3D) equipped with a reverse-phase column (Waters, Atlantis T3-3 µm (3.0 × 100 mm)). The temperature of the column oven was set to 40°C. A mixed solvent of water/methanol/tetrahydrofuran was used as the mobile phase, and the flow rate was 0.45 mL/min. The concentration of BHT in the sample was calculated using a calibration curve prepared from the peak area values of a standard sample of known concentration and the peak area values and dilution factor of the sample to be measured.

### [Manufacturing Example]

Radical polymerizable monomers having the following structures used in the examples were manufactured by the following methods. Note that "Me" in the drawings refers to a methyl group.

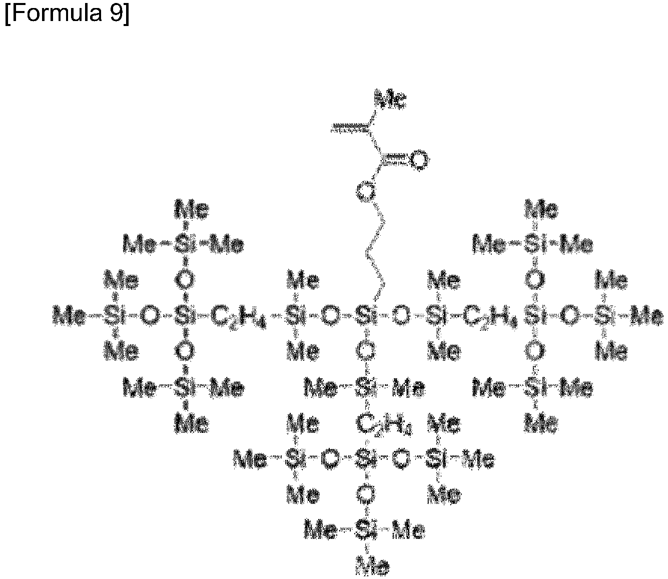

688 g of 1,1,1,5,5,5-hexamethyl-3-[(trimethylsilyl)oxy]-3-vinyltrisiloxane and 0.12 g of a toluene solution containing 5 mass% of a 1,3-diethenyl-1,1,3,3-tetramethyldisiloxane platinum complex (hereinafter referred to as platinum catalyst) were placed in a flask. After heating the liquid to 70°C, 136 g of methacryloxypropyl tris(dimethylsiloxy)silane solution containing 500 ppm by mass BHT was added dropwise over 2 hours while maintaining the liquid temperature at 70°C. The liquid temperature was maintained at 70°C for 2 hours and then at 120°C for 2 hours. Next, after performing a single distillation at 120°C and 1 kPa for 5 hours, the liquid in the flask was collected. The BHT concentration in the resulting solution was measured to be 160 ppm by mass.

### [Manufacturing Monomer Composition]

The device shown in FIG. 1 was used as a separation device for the polymerization inhibitor in order to manufacture the monomer compositions in Examples 1 to 5.

An end 17 of a first plastic tube was placed in a beaker 18 containing a magnetic stirrer, and another end 16 was connected to a suction port of a diaphragm pump 14. An end 15 of a second plastic tube was connected to an outlet of the diaphragm pump, and another end 13 was connected to an inlet of an activated charcoal immobilized filter 11 (SC050X AKJ from Osaka Gas Chemicals Co., Ltd.: activated carbon loading capacity: 1.45 g). An end 12 of a third plastic tube was connected to an outlet of the filter, and another end 19 was placed above the beaker 18.

### [Example 1]

Using the device shown in FIG. 1, 145 g of the radical polymerizable monomer manufactured by the manufacturing example was placed in a beaker and stirred at room temperature using the magnetic stirrer. The ratio of activated carbon mass to liquid mass was 0.01. The liquid in the beaker was supplied to the filter at a flow rate of 5.6 g/min (3.86 g/(min.g-AD)) and the discharged liquid was returned to the beaker and circulated. The circulation frequency, calculated as the circulation flow rate divided by the total liquid volume, was 2.3 times/hour. After 3 hours of circulation, a 1 g sample was taken from the beaker and analyzed for BHT concentration. The BHT concentration was 61.6 ppm by mass.

### [Example 2]

The same method as in Example 1 was used, except that 290 g of radical polymerizable monomer was used. In other words, the ratio of the activated carbon mass to the liquid mass was 0.005 and the circulation frequency was 1.2 times/hour. The BHT concentration was 128.6 ppm by mass.

### [Example 3]

The same method as in Example 2 was used, except that the circulation flow rate was set at 11.0 g/min (7.59 g/(min g-AD)). In other words, the ratio of the activated carbon mass to the liquid mass was 0.005 and the circulation frequency was 2.3 times/hour. The BHT concentration was 57.7 ppm by mass.

### [Example 4]

The same method as in Example 3 was used, except that the circulation time was set to 8 hours. In other words, the ratio of the activated carbon mass to the liquid mass was 0.005 and the circulation frequency was 2.3 times/hour. The BHT concentration was 31.1 ppm by mass.

### [Example 5]

The same method as in Example 4 was used, except that the temperature of the liquid in the beaker was maintained at 40°C using a thermostatic chamber. In other words, the ratio of the activated carbon mass to the liquid mass was 0.005 and the circulation frequency was 2.3 times/hour. The BHT concentration was 41.1 ppm by mass.

### [Example 6]

40 g of a radical polymerizable monomer produced in accordance with the manufacturing example and 8.1 g of activated alumina particles (Union Showa ST-1000) were placed in a beaker and stirred for 6 hours at room temperature using the magnetic stirrer. The ratio of activated alumina mass to liquid mass was 0.20. A 1 g sample of the liquid in the beaker was taken to measure the BHT concentration, and the BHT concentration was found to be 49.9 ppm by mass.

### [Example 7]

40 g of a radical polymerizable monomer produced in accordance with the manufacturing example and 4.0 g of activated alumina particles (Union Showa ST-1000) were placed in a beaker and stirred for 6 hours at room temperature using a magnetic stirrer. The ratio of activated alumina mass to liquid mass was 0.10. A 1 g sample of the liquid in the beaker was taken to measure the BHT concentration, and the BHT concentration was found to be 104.4 ppm by mass.

### [Comparative Example 1]

300 g of a radical polymerizable monomer produced in accordance with the manufacturing example was placed in a flask, depressurized with a vacuum pump to 2.0 kPa, and then heated with a mantle heater until the liquid temperature reached 125°C. After 3 hours of single distillation under these conditions, a 1 g sample of the liquid in the flask was collected and the BHT concentration was measured. The BHT concentration was 145.2 ppm by mass.

### [Comparative Example 2]

The same method as Comparative Example 1 was used, except that the duration of the single distillation was 6 hours. The BHT concentration was 139.2 ppm by mass.

### [Comparative Example 3]

The same method as Comparative Example 1 was used, except that the duration of the single distillation was 9 hours. The BHT concentration was 134.2 ppm by mass.

Table 1 Test conditions and BHT concentrations for Examples 1 through 5

**[Table 1]**

| | Units | Example 1 | Example 2 | Example 3 | Example 4 | Example 5 |
|---|---|---|---|---|---|---|
| Adsorption medium | | Activated charcoal immobilized filter | | | | |
| Ratio of adsorbent material mass to liquid mass | - | 0.01 | 0.005 | 0.005 | 0.005 | 0.005 |
| Circulation frequency | Times / h | 2.3 | 1.2 | 2.3 | 2.3 | 2.3 |
| Circulation time | Time | 3 | 3 | 3 | 8 | 8 |
| Liquid temperature | °C | Room temperature | Room temperature | Room temperature | Room temperature | 40 |
| BHT concentration | ppm by mass | 61.6 | 128.6 | 57.7 | 31.1 | 41.1 |

Table 2 Test conditions and BHT concentrations for Examples 6 and 7

**[Table 2]**

| | Units | Example 6 | Example 7 |
|---|---|---|---|
| Adsorption medium | - | Activated alumina particles | |
| Ratio of adsorbent material mass to liquid mass | - | 0.20 | 0.10 |
| Stirring time | Time | 6 | 6 |
| Liquid temperature | °C | Room temperature | Room temperature |
| BHT concentration | ppm by mass | 49.9 | 104.4 |

Table 3 Test conditions and BHT concentrations for Comparative Examples 1 through 3

**[Table 3]**

| | Units | Example 1 | Example 2 | Example 3 |
|---|---|---|---|---|
| Single distillation time | Time | 3 | 6 | 9 |
| BHT concentration | ppm by mass | 145.2 | 139.2 | 134.2 |

As can be seen from Tables 1 through 3, which summarize the results of each example, the method of the present invention makes it possible to obtain a monomer composition in which the total concentration of the polymerization inhibitor relative to the total mass of the radical polymerizable monomer and polymerization inhibitor is 130 ppm by mass or less. On the other hand, the single distillation method used in the comparative examples resulted in BHT concentrations exceeding 130 ppm by mass, even after prolonged treatment.

### [DESCRIPTION OF THE REFERENCE NUMERALS]

11 Activated charcoal immobilized filter
12 End of third plastic tube
13 Other end of second plastic tube
14 Diaphragm pump
15 End of second plastic tube
16 Other end of first plastic tube
17 End of first plastic tube
18 Beaker
19 Other end of third plastic tube

## Claims

1. A monomer composition, comprising: a radical polymerizable monomer having a radical polymerizable organic group and an organosilicon-containing organic group in a molecule, wherein total concentration of a polymerization inhibitor based on a total mass of the radical polymerizable monomer and the polymerization inhibitor is 130 ppm by mass or less.

2. The monomer composition according to claim 1, wherein the organosilicon-containing organic group in the radical polymerizable monomer is selected from a carbosiloxane dendrimer structure and a branched or linear siloxane structure.

3. The monomer composition according to claim 1 or claim 2, wherein
the radical polymerizable monomer is expressed by the following general formula (1): 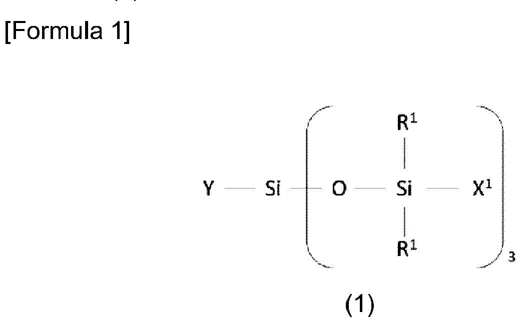 {where
Y is a radical polymerizable organic group,
R¹ is an alkyl group, an aryl group or a trimethylsiloxy group;
X¹ is a silylalkyl group expressed by the following general formula (2) when i = 1: 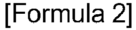 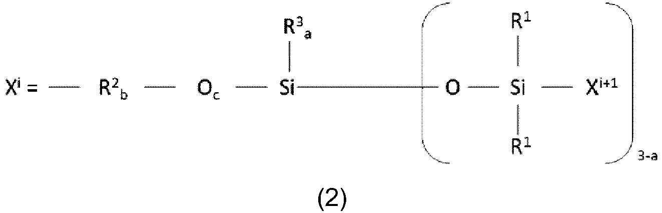 (where
R¹ is the same group as defined for general formula (1);
R² is an alkylene group with 2 to 10 carbon atoms;
R³ is a group selected from the group consisting of alkoxy groups, hydroxyl groups, alkyl groups, aryl groups, and trimethylsiloxy groups;
Xⁱ⁺¹ is a group selected from the group consisting of hydrogen atoms, alkyl groups with 1 to 10 carbon atoms, aryl groups, and the above silylalkyl groups, where i is an integer from 1 to 10 indicating the hierarchy of the silylalkyl group;
a is an integer from 0 to 3;
b and c are 0 or 1.)}.

4. The monomer composition according to any one of claims 1 to 3, wherein the radical polymerizable organic group includes a (meth)acryloyl group.

5. The monomer composition according to any one of claims 1 to 4, wherein the polymerization inhibitor includes one or more selected from hindered phenol-based polymerization inhibitors, hydroquinone-based polymerization inhibitors, and catechol-based polymerization inhibitors.

6. The monomer composition according to any one of claims 1 to 5, wherein the polymerization inhibitor includes one or more selected from dibutylhydroxytoluene (BHT), hydroquinone monomethyl ether (MEHQ), and 4-tert-butylcatechol (TBC).

7. The monomer composition according to any one of claims 1 to 6, wherein a total concentration of the polymerization inhibitor is 90 ppm by mass or less.

8. The monomer composition according to any one of claims 1 to 7, which is used for a cosmetic raw material.

9. A method for manufacturing the monomer composition according to any one of claims 1 to 8, comprising a step of bringing a liquid containing a radical polymerizable monomer into contact with an adsorbent material selected from the group consisting of activated carbon and alumina.

10. The manufacturing method according to claim 9, comprising a step of continuously supplying a liquid containing the radical polymerizable monomer to a fixed bed of the adsorbent material.

11. The manufacturing method according to claim 10, further comprising a step of circulating the liquid containing the radical polymerizable monomer by resupplying the outlet liquid of the fixed bed of the adsorbent material to the inlet.

12. The manufacturing method according to any one of claims 9 to 11, further comprising a step of adding a polymerization inhibitor other than BHT, MEHQ, and TBC after bringing the adsorbent material into contact with the liquid containing the radical polymerizable monomer.

13. Cosmetic material or cosmetic raw material, comprising the monomer composition according to any one of claims 1 to 8.
